(19) Europäisches Patentamt
European Patent Office
Office européen des brevets

(11) Publication number: **0 017 102**
**A1**

(12) # EUROPEAN PATENT APPLICATION

(21) Application number: 80101496.0

(22) Date of filing: 21.03.80

(51) Int. Cl.$^3$: **A 61 K 31/615**

(30) Priority: 22.03.79 US 22657

(43) Date of publication of application:
15.10.80 Bulletin 80/21

(84) Designated Contracting States:
AT BE CH DE FR GB IT LU NL SE

(71) Applicant: MERCK & CO. INC.
126, East Lincoln Avenue P.O. Box 2000
Rahway, New Jersey 07065(US)

(72) Inventor: Hirschmann, Ralph F.
740 Palmer Place
Blue Bell Pennsylvania 19422(US)

(72) Inventor: Kuehl, jr., Frederick A.
47 Wigwam Road
Locust New Jersey 07760(US)

(74) Representative: Abitz, Walter, Dr.-Ing. et al,
Abitz, Morf, Gritschneder P.O. Box 86 01 09
D-8000 München 86(DE)

(54) **Composition containing a phenyl benzoic acid compound and acetaminophen combination for the treatment of pain and inflammation and process for preparing the same.**

(57) A drug combination for more effective treatment of pain and inflammation comprising (a) a halogenated phenyl benzoic acid compound and (b) acetaminophen. The combination exhibits more rapid onset of action and more extended action, and provides more safety and fewer side effects than either ingredient alone.

EP 0 017 102 A1

Croydon Printing Company Ltd.

TITLE OF THE INVENTION

COMPOSITION CONTAINING A PHENYL BENZOIC ACID COMPOUND AND ACETAMINOPHEN COMBINATION FOR THE TREATMENT OF PAIN AND INFLAMMATION AND PROCESS FOR PREPARING THE SAME

BACKGROUND OF THE INVENTION:

Field of the Invention:

The present invention relates to a novel drug combination for more effective treatment of pain and inflammation.

Particularly, the present invention is concerned with the combination of (a) a phenyl benzoic acid compound, and (b) acetaminophen.

Pain, along with erythema and edema, is one of the three major symptoms of acute inflammation. Further, it is known that pain, inflammation, and prostaglandins are interrelated.

It is currently believed that prostaglandins (PGs) are released peripherally at a site of local inflammation, and act to sensitize pain receptors to the action of pain-inducing mediators, whatever their origin. Not only the primary prostaglandins ($PGE_2$ and $PGE_{2\alpha}$), but the endoperoxide intermediates ($PGG_2$ and $PGH_2$) as well, are involved. In fact, all of the oxygenation products resulting from the interaction of arachidonic

acid (AA) with prostaglandin synthetase (cyclo-oxygenase) must be considered potential pain-inducing agents. Thus, aspirin and other nonsteroidal anti-inflammatory agents (NSAI) have analgesic and anti-inflammatory activity due to their ability to depress the synthesis of prostaglandins by acting on the cyclo-oxygenase.

It is also currently believed that a destructive free radical oxidant released in the conversion of $PGG_2$ to $PGH_2$ plays a causal role in inflammatory processes, as well as in pain.

The phenyl benzoic acid compounds of the drug combination of the present invention are good prostaglandin cyclo-oxygenase inhibitors, and have a slight ability to neutralize the destructive oxidant as well. Acetaminophen, on the other hand, while it has no significant cyclooxygenase inhibiting activity, is a potent scavenger of the oxidative radical.

Surprisingly, the novel drug combination of the present invention possesses improved analgesia in the form of both more rapid onset of action, as well as in prolonged action.

Reference is made to the following for a more detailed discussion of the above principles: Kuehle and Egan, "Prostaglandins and Related Mediators in Pain", XIV Congress of Rheumatology, San Francisco, California, June 29, 1977; and Kuehl et al. Biochemical Aspects of Prostaglandins and Thromboxanes, Academic Press, pp. 55-74 (1977).

Brief Description of the Prior Art

The phenyl benzoic acid compounds employed in the present invention are known analgesic and anti-inflammatory compounds. See U.S. Patent Nos. 3,674,870 and 3,714,226. And, acetaminophen is a known analgesic agent. However, the combination is novel and the more effective treatment of pain and inflammation obtained therewith was wholly unexpected.

Acetaminophen esters of phenyl benzoic acid compounds, useful as analgesic and anti-inflammatory agents, are also known. See U.S. Patent No. 3,892,769. However, these esters represent 1:1 molar ratios of the phenyl benzoic acid and acetaminophen components of the basic molecule; whereas, in the physical mixture combination of the present invention, the acetaminophen is required to be present in several times the amount of the phenyl benzoic acid compound employed.

A eutectic mixture consisting of 63% aspirin and 37% acetaminophen is described in Japanese Patent No. 18,232; and a simple mixture of 10 grains of aspirin and 10 grains of acetaminophen is described in U.S. Patent No. 4,049,803. While these combinations claim to produce increased blood levels of aspirin in the blood, they do not suggest the novel drug combination of the present invention.

SUMMARY OF THE INVENTION:

In accordance with the present invention there is provided a novel drug combination for more effective treatment of pain and inflammation comprising (a) from 0.5 to 50.0% by weight of a phenyl benzoic acid compound, and (b) from 50.0 to 99.5% by weight of acetaminophen. Preferably, from 6.0 to 50.0% by weight of the phenyl benzoic acid compound and from 50.0 to 94.0%

by weight of the acetaminophen are employed.

The phenyl benzoic acid compounds utilized in the novel drug combination of the present invention are selected from compounds of the general formula:

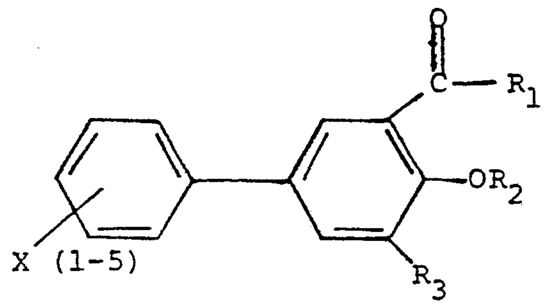

(I)

wherein X (1-5) is halo, such as fluoro or chloro, but especially fluoro; X being on one or more of the phenyl carbon atoms;

$R_1$ is selected from the group consisting of hydroxy, phenoxy diloweralkylamino (such as dimethylamine), diloweralkylamino loweralkoxy (such as diethylaminoethoxy);

$R_2$ is selected from the group consisting of hydrogen and lower alkanoyl (such as acetyl, propionyl and butyryl); and

$R_3$ is selected from the group consisting of hydrogen and methyl.

Also included are the pharmaceutically non-toxic salts of the acids of the compounds of Formula I such as the ammonium, alkali metal (such as sodium or potassium); alkaline earth metals (such as calcium, barium or magnesium); amine; aluminum; iron; choline; glucosamine; S-methyl methonine salts, piperazine, diloweralkylamino lower alkanol, chloroquine and hydroxy chloroquine; the anhydride of said acids, the mixed anhydrides of said acids and 2-acetoxy benzoic acid.

Especially preferred phenyl benzoic acid compounds are those wherein;

$R_1$ is hydroxy,

$R_2$ is hydrogen or acetyl,

$R_3$ is hydrogen, and

X is fluoro,

X being on any position of the phenyl moiety when X is one fluoro group, but particularly on the 4'-position; and where X represents two fluoro groups, particularly on the 2'-and 4'-positions.

Representative phenyl benzoic acid compounds are as follows:

2-hydroxy-5-(2',4'-difluorophenyl)benzoic acid;

2-acetoxy-5-(2',4'-difluorophenyl)benzoic acid;

2-hydroxy-5-(2'-fluorophenyl)benzoic acid;

2-hydroxy-5-(4'-fluorophenyl)benzoic acid;

2-hydroxy-5-(3'-fluorophenyl)benzoic acid;

2-hydroxy-5-pentafluorophenyl benzoic acid;

2-hydroxy-3-methyl-5-(2',4'-difluorophenyl)benzoic acid;

2-hydroxy-5-(2'-chloro-4'-fluorophenyl)benzoic acid;

N,N-dimethyl-5-(2',4'-difluorophenyl)salicylamide;

β-diethylaminoethyl-5-(2',4'-difluorophenyl)salicylate;

phenyl-5-(2',4'-difluorophenyl)salicylate;

aluminum-2-acetoxy-5-(2',4'-difluorophenyl)-benzoate salt;

aluminum-2-hydroxy-5-(2',4'-difluorophenyl)-benzoate salt;

choline-2-acetoxy-5-(2',4'-difluorophenyl)-benzoate salt;

choline-2-hydroxy-5-(2',4'-difluorophenyl)-benzoate salt;

sodium-2-acetoxy-5-(2',4'-difluorophenyl)-benzoate salt;

sodium-2-hydroxy-5-(2',4'-difluorophenyl)-benzoate salt;

2-acetoxy-5-(pentafluorophenyl)-benzoic acid;

β-diethylaminoethyl-2-hydroxy-5-(2',4'-difluorophenyl)-benzoate;

β-diethylaminoethyl-2-acetoxy-5-(2',4'-difluorophenyl)-benzoate.

Acetaminophen is represented by the following formula:

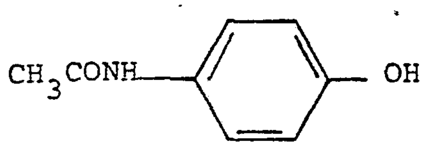

(II)

The treatment of pain and inflammation in accordance with the method of the present invention is accomplished by orally administering to patients in need of such treatment a composition having as its active ingredient a mixture of from 0.5 to 50.0 % by weight of a compound of Formula I, particularly the especially preferred compounds, and from 50.0 to 99.5 % by weight of the compound of Formula II, in a non-toxic pharmaceutically acceptable carrier, preferably in tablet or capsule form.

The non-toxic pharmaceutical carrier may be for example, either a solid or a liquid. Exemplary of solid carriers are lactose, corn starch, gelatin, talc, stearic acid, magnesium stearate, terra alba, sucrose, agar, pectin, cab-o-sil, and acacia. Exemplary of liquid carriers are peanut oil, olive oil, sesame oil and water. Similarly, the carrier or diluent may include a time delay material such as glyceryl monostearate or glyceryl distearate alone or with a wax.

Several pharmaceutical forms of the therapeutically useful compositions can be used. For example, if a solid carrier is used, the compositions may take the form of tablets, capsules, powders, troches or lozenges, prepared by standard pharmaceutical techniques. If a liquid carrier is used, the preparation may be in the form of a soft gelatin capsule, a syrup or a liquid supsension.

The active compounds of Formulas I and II and of the compositions of this invention are present in an amount sufficient to treat pain and inflammation, that is to reduce pain and inflammation. Advantageously, the composition will contain the active ingredient, namely, the compounds of Formulas I and II in an amount of from about 1 mg. to 140 mg. per kg. body weight per day (50 mg. to 10 g. per patient per day), preferably from about 2 mg. to 70 mg. per kg. body weight per day (100 mg. to 5 g. per patient per day).

The method of treatment of this invention comprises internally administering to a patient (animal or human), compounds of Formulas I and II, particularly including an especially preferred compound of Formula I admixed with a non-toxic pharmaceutical carrier such as exemplified above. The mixture of the compounds of Formulas I and II will be present in an amount of from 1 mg. to 140 mg./kg. body weight per day, preferably from about 2 mg. to about 70 mg. per kilogram body weight per day and especially from 4 mg. to 10 mg./kg. body weight per day.

The most rapid and effective anti-inflammatory effect is obtained from oral administration of a daily dosage of from about 4 to 10 mg./kg. day. It should be understood, however, that although preferred dosage ranges are given, the dose level for any particular patient depends upon the activity of the specific compound employed. Also many other factors that modify the actions of drugs will be taken into account by those skilled in the art in the therapeutic use of medicinal agents, particularly those of Formulas I and II, for example, age, body weight, sex, time of administration, route of administration, rate of excretion, drug combination, reaction sensitivities and severity of the particular disease.

The more effective treatment of pain achieved with the novel drug combination of the present invention was evaluated in a bioassay designed to detect the analgesic activity of the combination. Since analgesic tests using intact animals are not sensitive enough to detect the analgesic activity of nonsteroidal anti-inflammatory compounds, the analgesic bioassay employed utilized inflammatory sensitization procedures, specifically adjuvant arthritis. Adjuvant arthritis induced in rats, the animals used in the bioassay, resembles rheumatoid arthritis in man with respect to the manifestation of inflammation and hyperalgesia. When the affected joints of rats with adjuvant arthritis are subjected to a gentle manipulation, preferably by extending the joint, the rats respond by squeaking, which is a definite and reproducible sign of algesia. The ability of the tested compounds to suppress this squeaking response was an accurate measure of the analgesic activity of the tested compounds. Further, the anti-inflammatory activity of the tested compounds played only a minor role in the joint extension

analgesic activity of the compounds, because the adjuvant arthritis lesions were relatively tolerant to the anti-inflammatory action, and because the analgesic activity of the compounds could be demonstrated at very low doses.

In accordance with the adjuvant arthritis bioassay used to evaluate the analgesic activity of the combinations of compounds of the present invention, young adult Sprague-Dawley rats of either sex are rendered hyperalgesic by injection of dessicated Mycobacterium-butyricum suspended in light mineral oil (Freund's adjuvant). The mycobacteria are ground in a mortar, suspended in the oil, and sterilized in an autoclave. Each animal receives 0.5 mg of the bacteria in 0.1 ml of oil, injected subcutaneously through a 21-gauge needle into the distal third of the tail.

The animals can subsequently be used in two ways for testing the analgesic action of drugs. The day after the injection of adjuvant, the tail is found to be sensitive to touch. Approximately 16 hours after the injection, the experimenter tests the animals for the presence of hyperalgesia. This is done by holding the animal in one hand. With the thumb and forefinger of the other hand, the observer applies gentle pressure to the tail about 2.5 cm proximal to the point where the needle has been inserted. This gentle pressure is repeated at 5-sec intervals. If the rat endures five such gentle presses without emitting more than one "squeak", analgesia is considered to be present and a score of + is assigned. Two or more squeaks earns a rating of 0, or no analgesia. Before being assigned to an experiment, animals are screened and only those with the 0 rating are used.

The hyperalgesic animals are assigned randomly to drug treatments and one of the treatments is always the vehicle without the drug (placebo group). When testing

the animals, the observer does not know which treatment an individual animal has received. Presence or absence of analgesia is determined at one-hour intervals for a total of six (6) hours after administration of the drug and one can calculate the $ED_{50}$, that is the dose required to produce analgesia in one-half of the animals. This entire procedure may be referred to as "tail pinch" technique. Alternatively, an apparatus for applying pressure to the tail may be employed and the "squeak threshold" determined before and after drug.

The adjuvant-injected animals, if left undisturbed, develop severe arthritis which becomes especially manifest in about two weeks. These rats also exhibit hyperalgesia and if the hind feet are gently manipulated, a "squeak" response is readily elicited in a manner similar to that described above by pinching the tail the day after adjuvant injection. This "foot bend" technique yields drug responses essentially identical with those obtained by the "tail pinch" procedure.

The adjuvant arthritis analgesic bioassay described above correlates with analgesic activity in humans, since compounds known to be clinically active, including indomethacin, phenylbutazone, ibufenac, and aspirin, give positive, dose-dependent, reproducible results in this assay. For a further description of this bioassay and its significance, reference is made to Kuzuma and Kawai, "Evaluation of Analgesic Agents in Rats with Adjuvant Arthritis", Chem. Pharm. Bull., 23 (6) 1184-1191 (1975).

The data obtained from the adjuvant arthritis analgesic bioassay is summarized in the tables of values below.

TABLE I.

| Compound | Dose (mg./kg.) | No. of Rats per Dose | Number of Rats with Analgesia (hours after Administration 1 | 2 | 3 | 4 | 5 | 6 |
|---|---|---|---|---|---|---|---|---|
| APAP ☆ | 100 | 8 | 0 | 1 | 1 | 0 | 0 | 0 |
| | 200 | 8 | 2 | 4 | 4 | 4 | 2 | 1 |
| | 400 | 8 | 6 | 7 | 6 | 5 | 2 | 1 |
| Diflunisal ☆☆ | 4 | 8 | 0 | 2 | 1 | 0 | 0 | 0 |
| | 8 | 8 | 0 | 1 | 3 | 1 | 0 | 0 |
| | 16 | 8 | 0 | 4 | 6 | 3 | 2 | 1 |
| APAP + Diflunisal | 200+4 | 8 | 4 | 5 | 4 | 4 | 1 | 0 |
| | 200+8 | 8 | 5 | 7 | 6 | 4 | 3 | 3 |
| | 200+16 | 8 | 3 | 4 | 5 | 6 | 5 | 2 |
| APAP + Diflunisal | 200+2 | 8 | 5 | 5 | 3 | 2 | 2 | 1 |
| | 200+4 | 8 | 6 | 5 | 6 | 4 | 4 | 1 |
| | 200+8 | 8 | 5 | 7 | 6 | 6 | 5 | 4 |
| APAP | 25 | 10 | 1 | 0 | 0 | 0 | 0 | 0 |
| | 50 | 10 | 1 | 0 | 0 | 0 | 0 | 0 |
| | 100 | 10 | 3 | 2 | 0 | 0 | 0 | 0 |
| Diflunisal | 2 | 10 | 2 | 2 | 2 | 0 | 0 | 0 |
| | 4 | 10 | 0 | 1 | 0 | 0 | 1 | 0 |
| | 8 | 10 | 4 | 4 | 5 | 3 | 2 | 1 |
| APAP + Diflunisal | 25+2 | 10 | 1 | 2 | 1 | 1 | 0 | 0 |
| | 50+2 | 10 | 4 | 5 | 2 | 1 | 1 | 1 |
| | 100+2 | 10 | 3 | 5 | 3 | 0 | 0 | 0 |
| APAP + Diflunisal | 25+4 | 10 | 2 | 2 | 2 | 2 | 1 | 0 |
| | 50+4 | 10 | 1 | 1 | 0 | 0 | 0 | 0 |
| | 100+4 | 10 | 3 | 6 | 6 | 2 | 2 | 0 |

☆ APAP = acetaminophen

☆☆ Diflunisal = 2-hydroxy-5-(2',4'-difluorophenyl)benzoic acid

TABLE I (con'd)

| Compound | Dose (mg./kg.) | No. of Rats per Dose | Number of Rats with Analgesia (Hours after Administration 1 | 2 | 3 | 4 | 5 | 6 |
|---|---|---|---|---|---|---|---|---|
| APAP + Diflunisal | 25+8 | 10 | 4 | 5 | 3 | 3 | 2 | 0 |
| | 50+8 | 10 | 4 | 5 | 3 | 3 | 2 | 1 |
| | 100+8 | 10 | 5 | 8 | 8 | 5 | 1 | 0 |
| APAP | 100 | 8 | 1 | 1 | 0 | 0 | 0 | 0 |
| | 200 | 8 | 3 | 4 | 4 | 4 | 1 | 0 |
| | 400 | 8 | 4 | 7 | 6 | 4 | 3 | 1 |
| Diflunisal | 5 | 8 | 0 | 1 | 2 | 1 | 0 | 0 |
| | 10 | 8 | 0 | 1 | 4 | 4 | 2 | 0 |
| | 20 | 8 | 1 | 4 | 7 | 7 | 5 | 4 |
| APAP + Diflunisal | 50+2.5 | 8 | 0 | 1 | 2 | 0 | 0 | 0 |
| | 100+5 | 8 | 1 | 3 | 3 | 3 | 1 | 0 |
| | 200+10 | 8 | 5 | 7 | 7 | 6 | 5 | 3 |

In order that the above data might be better understood, portions of that data have been illustrated in the drawing accompanying this description.

FIG. 1 is a graph of the analgesia data for 50 mg./kg. of acetaminophen and 2 mg./kg. of diflunisal, as well as a mixture of these. These doses are relatively small doses.

FIG. 2 is a graph of the analgesia data for relatively moderate doses, i.e., for 200 mg./kg. of acetaminophen and 8 mg./kg. of diflunisal, as well as a mixture of these.

The data above, and particularly the graphs of that data, clearly show the improved analgesia obtained with the combination of phenyl benzoic acid compounds and acetaminophen, over either separate component alone. That improved analgesia occurs in the form of both more rapid

onset of action, as well as in prolonged action. Also, the improved analgesia occurs over basically the entire range of proportionate amounts of the phenyl benzoic acid compound and acetaminophen in the combination. The improved analgesia is particularly clear where the combination of phenyl benzoic acid compound and acetaminophen provides analgesic activity at dose levels where either one or both of the separate components alone, at the same dose level, provides no analgesic activity at all. Data showing this particular aspect of the improved analgesia obtained with the novel drug combination of the present invention is summarized in the table below, and is taken from the data set out in TABLE I above:

TABLE II.

| No. of Rats per Dose | Time after Drug (Hrs.) | Dose (mg./kg.) | | Percent of Rats with Analgesia | | |
|---|---|---|---|---|---|---|
| | | APAP | Diflunisal | APAP Alone | Diflunisal Alone | Combination |
| 10 | 4 | 25 | 2 | 0 | 0 | 10 |
| 10 | 1 | 25 | 4 | 10 | 0 | 20 |
| 10 | 4 | 50 | 2 | 0 | 0 | 10 |
| 10 | 2 | 50 | 2 | 0 | 20 | 50 |
| 8 | 5 | 100 | 5 | 0 | 0 | 12.5 |
| 8 | 3 | 100 | 5 | 0 | 25 | 37.5 |
| 8 | 4 | 100 | 5 | 0 | 10 | 30 |
| 10 | 3 | 100 | 4 | 0 | 0 | 60 |
| 10 | 4 | 100 | 4 | 0 | 0 | 20 |
| 10 | 3 | 100 | 8 | 0 | 50 | 80 |
| 10 | 4 | 100 | 8 | 0 | 30 | 50 |
| 8 | 6 | 200 | 10 | 0 | 0 | 30 |
| 8 | 6 | 200 | 8 | 12.5 | 0 | 30 |
| 8 | 1 | 200 | 4 | 25 | 0 | 50 |
| 8 | 1 | 200 | 8 | 25 | 0 | 82.5 |
| 8 | 5 | 200 | 8 | 25 | 0 | 37.5 |
| 8 | 1 | 200 | 10 | 37.5 | 0 | 82.5 |
| 8 | 1 | 200 | 16 | 25 | 0 | 37.5 |

The above data again clearly shows the more rapid onset of action and the prolonged action obtained with the novel drug combination of the present invention.

In order to further illustrate the more rapid onset of action achieved with the novel drug combination of the present invention, the following TABLE III presents data from observations at one (1) and (2) hours after administration of the test drug in the adjuvant arthritis analgesic bioassay described above.

TABLE III.

| Compound | Dose (mg./kg.) | No. of Rats per Dose | Number of Rats with Analgesia (Hours after Administration 1/2 | 1 | 2 | Total Positives |
|---|---|---|---|---|---|---|
| Diflunisal | 4 | 10 | - | 0 | 1 | 1 |
| APAP | 100 | 10 | - | 3 | 2 | 5 |
| APAP + Diflunisal | 25 + 4 | 10 | - | 2 | 2 | 4 |
| | 50 + 4 | 10 | - | 1 | 1 | 2 |
| | 100 + 4 | 10 | - | 3 | 6 | 9 |
| Diflunisal | 5 | 8 | 0 | 0 | 1 | 1 |
| | 10 | 8 | 0 | 0 | 1 | 1 |
| APAP | 200 | 8 | 2 | 2 | 3 | 7 |
| APAP + Diflunisal | 100 + 5 | 8 | 0 | 1 | 3 | 4 |
| | 200 + 10 | 8 | 3 | 5 | 7 | 15 |

Another aspect of the improved effectiveness of the novel drug combination of the present invention is the unexpectedly enhanced safety of the combination as compared to the safety of the separate components alone. Measurements of the relative safety of the components and of the combination were carried out using acute oral toxicity studies in female mice. Randomly selected female albino mice (Carworth CF-1) weighing from 18 to 24 grams, and 6 to 7 weeks old, were used. The $LD_{50}$ value for each drug was estimated from acute oral dose-response curves previously established. The following dose regimen was utilized:

| Group | Diflunisal | Acetaminophen |
|---|---|---|
| I | 100% $LD_{50}$ | 0 |
| II | 75% $LD_{50}$ | 25% $LD_{50}$ |
| III | 50% $LD_{50}$ | 50% $LD_{50}$ |
| IV | 25% $LD_{50}$ | 75% $LD_{50}$ |
| V | 0 | 100% $LD_{50}$ |

Both drugs were prepared as suspensions in 1% aqueous methylcellulose (100 cps.), diflunisal as a 2% concentration and acetaminophen as a 5% concentration. The preparations were administered by gastric intubation using a metal catheter attached to a springe. The various doses were administered randomly with 20 mice per group. Food was withheld one to two hours before and three hours following drug treatment. Water was available at all times. When the two drugs were administered,diflunisal was always given first. The surviving mice were observed closely on the day of treatment and daily thereafter for a total test period of 14 days. Body weights were taken pretest and on days 7 and 14. Results of the acute oral toxicity study are presented in the following table of data:

TABLE IV

| Group | Diflunisal %$LD_{50}$ | Diflunisal mg./kg. | Acetaminophen %$LD_{50}$ | Acetaminophen mg./kg. | No. Dead | No. Tested |
|---|---|---|---|---|---|---|
| I | 100 | 400 | 0 | 0 | 14 | 20 |
| II | 75 | 300 | 25 | 375 | 16 | 20 |
| III | 50 | 200 | 50 | 750 | 5 | 20 |
| IV | 25 | 100 | 75 | 1125 | 9 | 20 |
| V | 0 | 0 | 100 | 1500 | 16 | 20 |

WHAT IS CLAIMED IS:

1. In combination:

(a) from 0.5 to 50.0 % by weight of a phenyl benzoic acid compound of the general formula:

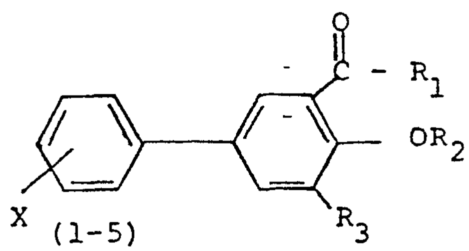

wherein

$X_{(1-5)}$ is halo; X being on one or more of the phenyl carbon atoms;

$R_1$ is selected from the group consisting of hydroxy, phenoxy, diloweralkylamino, and diloweralkylamino loweralkoxy;

$R_2$ is slected from the group consisting of hydrogen and lower alkanoyl; and

$R_3$ is selected from the group consisting of hydrogen and methyl;

and pharmaceutically acceptable, non-toxic salts thereof; and;

(b) from 50.0 to 99.5 % by weight of acetaminophen.

2. The combination of Claim 1 wherein the phenyl benzoic acid compound is 2-hydroxy-5-(2',4'-difluorophenyl) benzoic acid.

3. A pharmaceutical composition for treating pain and inflammation consisting of an effective amount of the combination

(a) from 0.5 to 50.0% by weight of a phenyl benzoic acid compound of the general formula:

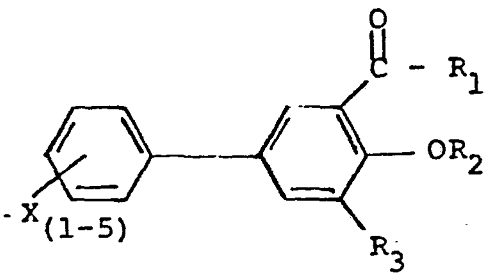

wherein

$X_{(1-5)}$ is halo; X being on one or more of the phenyl carbon atoms;

$R_1$ is selected from the group consisting of hydroxy, phenoxy, diloweralkylamino, and diloweralkylamino loweralkoxy;

$R_2$ is selected from the group consisting of hydrogen and lower alkanoyl; and

$R_3$ is selected from the group consisting of hydrogen and methyl;

and pharmaceutically acceptable, non-toxic salts thereof; and

(b) from 50.0 to 99.5% by weight of acetaminophen as an active ingredient; together with a pharmaceutically acceptable non-toxic carrier.

4. The composition of Claim 3 wherein the phenyl benzoic acid compound is 2-hydroxy-5-(2',4'-difluorophenyl) benzoic acid.

Claims for Austria

1. Process for preparing a pharmaceutical composition for treating pain and inflammation, characterized by mixing together an effective amount of

(a) from 0.5 to 50.0% by weight of a phenyl benzoic acid compound of the general formula

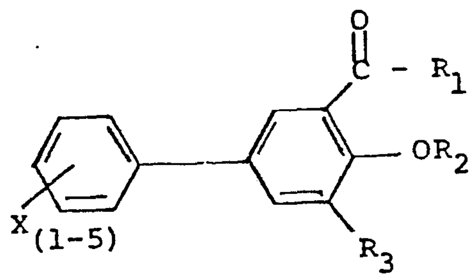

wherein

$X_{(1-5)}$ is halo; X being on one or more of the phenyl carbon atoms;

$R_1$ is selected from the group consisting of hydroxy, phenoxy, diloweralkylamino, and diloweralkylamino loweralkoxy;

$R_2$ is selected from the group consisting of hydrogen and lower alkanoyl; and

$R_3$ is selected from the group consisting of hydrogen and methyl;

and pharmaceutically acceptable, non-toxic salts thereof;

(b) from 50.0 to 99.5% by weight of acetaminophen as an active ingredient; and, if desired

(c) a pharmaceutically acceptable non-toxic carrier.

2. Process of Claim 1 wherein the phenyl benzoic acid compound is 2-hydroxy-5-(2',4'-difluorophenyl) benzoic acid.

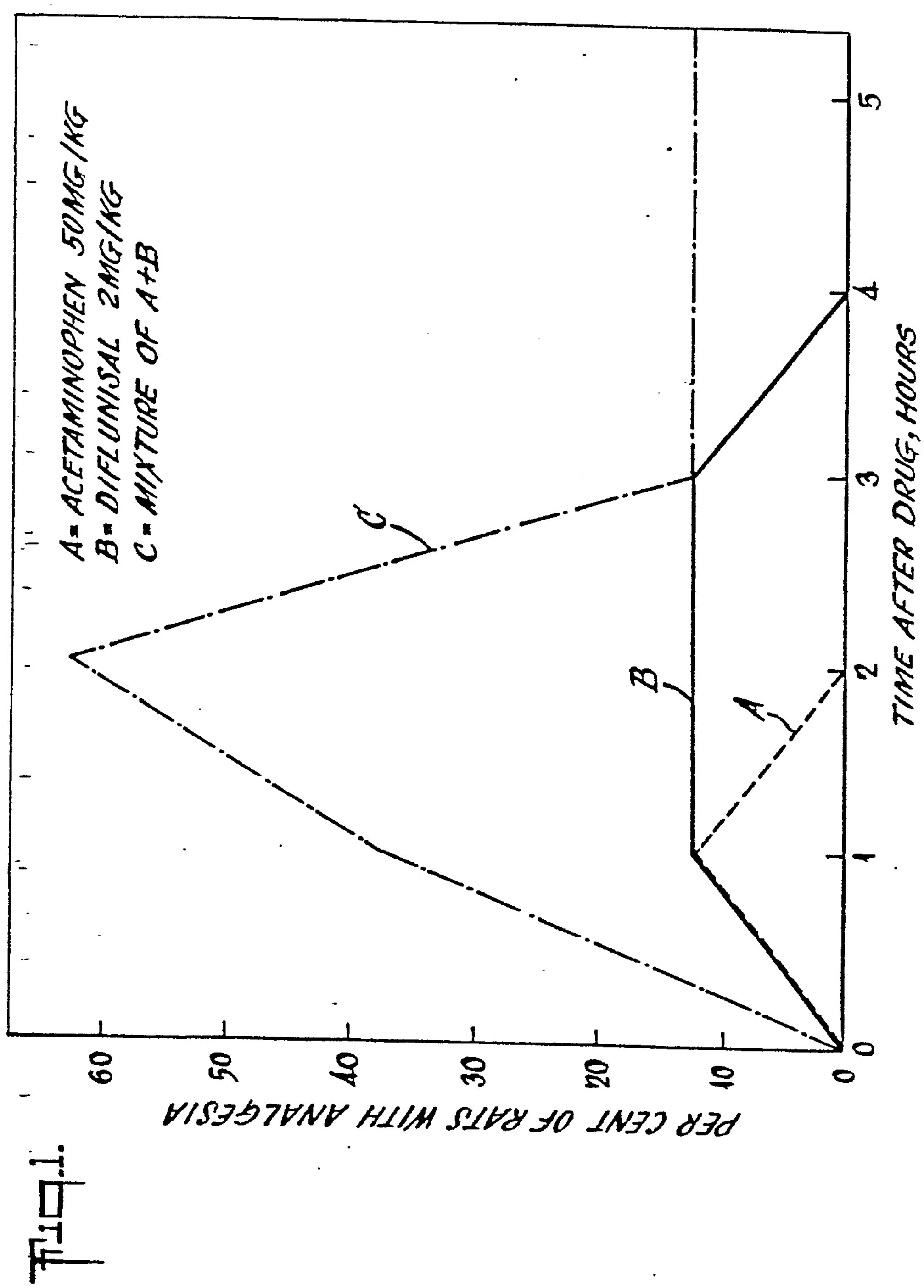

A= ACETAMINOPHEN 50MG/KG
B= DIFLUNISAL 2MG/KG
C= MIXTURE OF A+B
A
B
C
0
1
2
3
4
5
TIME AFTER DRUG, HOURS
0
10
20
30
40
50
60
PER CENT OF RATS WITH ANALGESIA


FIG. 1.

0017102



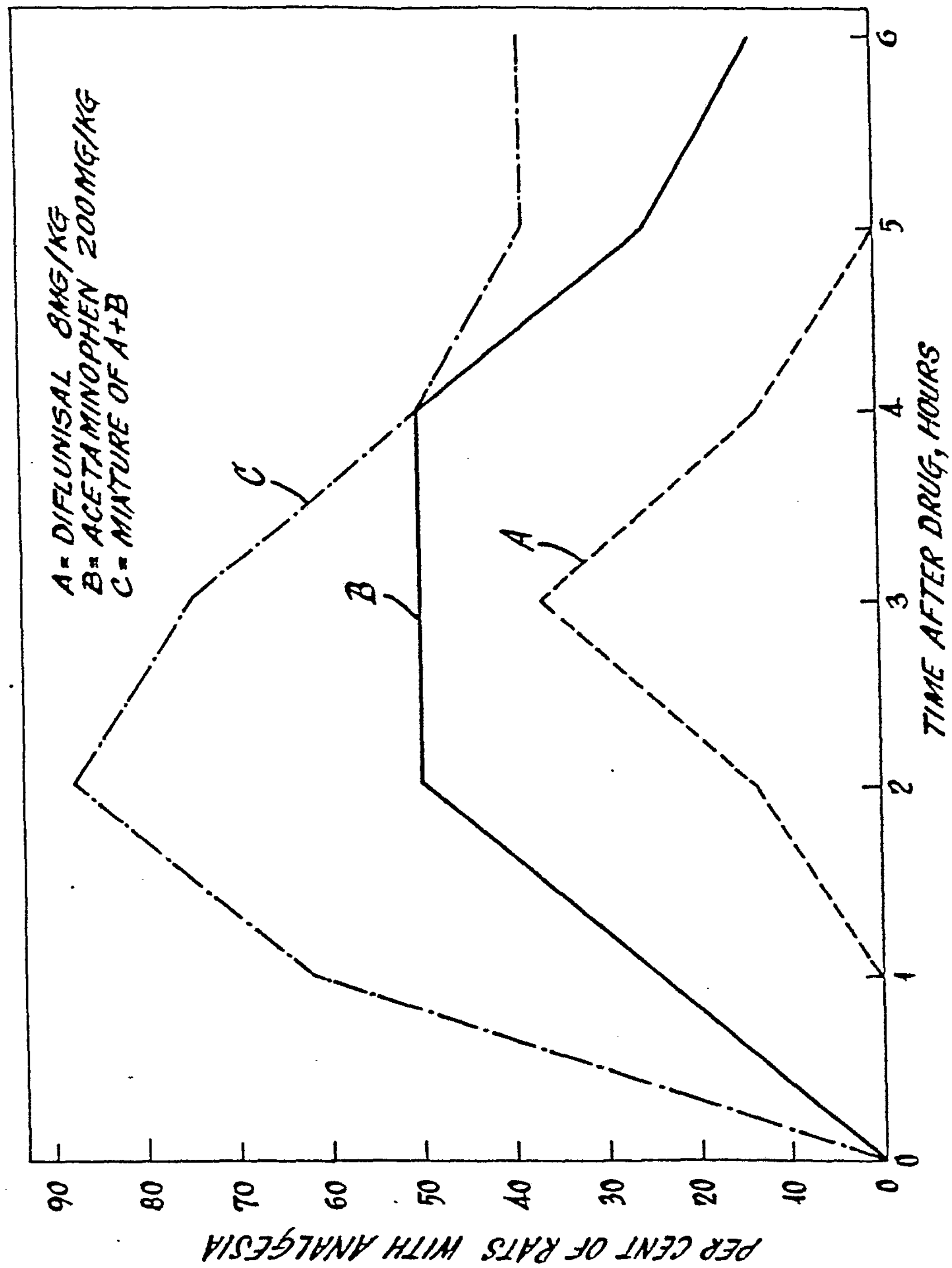


Fig. 2.

European Patent Office

# EUROPEAN SEARCH REPORT

Application number
EP 80 10 1496

## DOCUMENTS CONSIDERED TO BE RELEVANT

| Category | Citation of document with indication, where appropriate, of relevant passages | Relevant to claim |
|---|---|---|
| D | US - A - 3 892 769 (TSUNG-YING SHEN)<br>* Column 1, lines 1-35; column 6, line 63 - column 8, line 36; examples 4-8 *<br>-- | 1-4 |
| A | ROTE LISTE, 1961, Editio Cantor, Aulendorf/Württ, page 348<br>FENSUN: "Kindersuppositorien"<br>-- | 1 |
| A | UNLISTED DRUGS, vol. 23, July 1971, page 100c<br>Chatham, N.J., U.S.A.<br>"Paleva"<br>---- | 1 |

CLASSIFICATION OF THE APPLICATION (Int. Cl. [3])

A 61 K 31/615

TECHNICAL FIELDS SEARCHED (Int.Cl. [3])

A 61 K 31/615

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 25-06-1980 | BRINKMANN |

EPO Form 1503.1 06.78